Europäisches Patentamt

European Patent Office

Office européen des brevets

(11) Publication number : **0 512 849 A1**

# (12) EUROPEAN PATENT APPLICATION

(21) Application number : 92304155.2

(22) Date of filing : 08.05.92

(51) Int. Cl.⁵ : **A61K 31/73**

(30) Priority : **09.05.91 JP 135959/91**

(43) Date of publication of application :
**11.11.92 Bulletin 92/46**

(84) Designated Contracting States :
**DE FR GB IT**

(71) Applicant : **UNITIKA LTD.**
**No. 50, Higashihonmachi 1-chome**
**Amagasaki-shi Hyogo (JP)**

(72) Inventor : **Nakamura, Yuriko c/o Unitika Ltd.**
**23, Uji Kozakura Uji-shi**
**Kyoto (JP)**
Inventor : **Kifune, Koji c/o Unitika Ltd.**
**23, Uji Kozakura**
**Uji-shi Kyoto (JP)**
Inventor : **Tsurutani, Ryoichi c/o Unitika Ltd.**
**23 Uji Kozakura Uji-shi**
**Kyoto (JP)**
Inventor : **Tanimoto, Nobuyuki c/o Unitika Ltd.**
**23, Uji Kozakura Uji-shi**
**Kyoto (JP)**

(74) Representative : **Holmes, Michael John**
**Frank B. Dehn & Co. Imperial House 15-19**
**Kingsway**
**London WC2B 6UZ (GB)**

(54) **Use of deacylated chitin derivatives for the treatment of tumours of the haemopoietic system.**

(57)    The present invention relates to a therapeutic agent composed of chitin for the treatment of tumors of blood cells, and more particularly relates to a therapeutic agent suitable for malignant tumors of blood cells, especially for diseases such as adult T-cell leukemia (ATL) and Sézary's syndrome.
    The present invention provides a therapeutic agent for blood cells tumors, countaining deacetylated chitin, preferably chitin with deacetylation of not less than 30%. The agent preferably contains at least 0.3% by weight, of deacetylated chitin.

EP 0 512 849 A1

The present invention relates to a therapeutic agent composed of chitin for the treatment of tumors of blood cells, and more particularly relates to a therapeutic agent suitable for malignant tumors of blood cells, especially for diseases such as adult T-cell leukemia (ATL) and Sézary's syndrome.

Tumor is defined as normal somatic cells changing their biological characteristics by various tumor forming stimulations to exhibit excessive autonomous growth, and is classified into two types, namely benign and malignant. Tumors which exert strong effects on the body, markedly damaging functions of various organs, growing rapidly and readily metastasize or recur are called malignant tumors. Such malignant tumors (recently, malignant tumors in general are called cancer) can occur in various body tissues, and are classified according to the tissues from which the tumor cells are derived.

In particular, leukemia differs from other solid malignant tumors in that the tumor cells can proliferate and survive separately and individually to be distributed into all parts of the body including bone marrow. Conventionally, treatment of leukemia has been primarily by potent chemotherapy using large volumes of antitumor drugs. Although it is not impossible to produce cure by the conventional chemotherapy, but its probability is low at present. For this reason, bone marrow transplantation has been proposed as a therapy designed to actively attain cure. Bone marrow transplantation, however, has the disadvantage of not being feasible unless requirements such as the question of age, absence of complications in the recipient, and finding the HLA (human leukocyte antigen) compatible donors, are satisfied. In this respect, chemotherapy may be initiated immediately after diagnosis of leukemia for induction of remission. With the advent of a variety of antitumor agents such as busulfan, cyclophosphamide, mercaptopurine, hydroxyurea and interferon, and with the advancement in adjuvant therapies, chemotherapy of leukemia has become a possibility for obtaining a complete cure.

Meanwhile, antitumor agent containing chitin, chitin oligomer and chitosan oligomer have been proposed (Reference Public Bulletins JP-A-59-27826(1984) and JP-A-62-123123(1987)). Further, antitumor effect of deacetylated chitin has been recognized, but this has been taken as the effect of directly causing damages of tumor cells (Public Bulletin JP-A-59-27826(1984)), and its antitumor effect through immune reactivation has not been known to date. Furthermore, it has not been known to use deacetylated chitin particularly for the treatment of tumors of blood cells.

As chemotherapy often utilizes large volumes of powerful antitumor agents, many untoward side effects also appear. The most frequently observed side effect of antileukemic agents is nausea and vomiting. In addition, alopecia is an inevitable side effect.

Treatment of leukemia would not be possible if side effects are feared, however, it is desirable to reduce side effects as much as possible, as they are a great burden for the patients.

In this respect, an antitumor agent utilizing chitin exerts moderate effects and may be considered a therapeutic agent without side effect, as chitin is a naturally occurring polymer and its metabolic products are already existing in vivo.

The objective of the present invention is to provide a therapeutic agent with an excellent therapeutic effect against diseases mainly of blood cells tumors without the side effects that pose problems in the use of other antitumor agents.

The inventors of the present invention, as a result of their strenuous efforts in the research work designed to achieve the aforementioned objective, have found that deacetylated chitin has an excellent antitumor effect through reactivation of host immunity, and arrived at the present invention.

That is to say, the present invention pertains to a therapeutic agent containing deacetylated chitin for the treatment of tumors of blood cells.

Originally, chitin is poly(N-acetyl-D-glucosamine), which is derived from the external skeletons of crustacea and insects by removing ashes and proteins with hydrochloric acid and caustic soda, however, chitin as referred to in the present invention includes chitin derivatives with esterified, etherified, carboxymethylated, hydroxyethylated or O-ethylated -OH groups including -$CH_2OH$ groups of glucosamine residues.

Further, deacetylated chitin is to mean that aminoacetyl group of chitin is deacetylated to form amino group. It is preferably chitin with 30 to 100% deacetylation, more preferably with 50 to 95% deacetylation, and most preferably 60 to 85% deacetylation. In addition, deacetylated chitin contained in the antitumor agent of the present invention may be a salt formed by amino group and acids, for example, acetate, hydrochloride, sulfate and phosphate.

Deacetylation of chitin may be accomplished by a well known method of processing chitin with alkali. In this process, the degree of deacetylation can be readily adjusted through appropriate adjusting alkali concentrations, processing temperature and processing time.

The degree of deacetylation referred to herein is the value determined by the following method. Approximately 2g of sample is placed in 200ml of aqueous solution of 2N hydrochloric acid, and stirred for 30 minutes at room temperature, filtered through a glass filter to remove the aqueous solution of hydrochloric acid. The residue is placed in 200ml of methanol, and stirred for 30 minutes. This is again filtered through a glass filter,

EP 0 512 849 A1

then the residue is placed in 200ml of fresh methanol and stirred for 30 minutes. After repeating the washing with methanol four times, and the residue is air-dried and dried under reduced pressure. Approximately 0.2g of the residue is precisely weighed, placed in Erlenmeyer flask of 100ml capacity, to which 40ml of ion-exchanged water is added and the mixture stirred for 30 minutes. The solution is subjected to acid-base titration in 01N caustic soda solution using phenolphthalein as an indicator. The degree of deacetylation (A) is calculated from the following equation:

$$A\,(\%) \;=\; \frac{2.03 \times f \times b \times 10^{-2}}{a \,+\, 0.55 \times f \times b \times 10^{-2}} \times 100$$

where a is weight(g) of the sample, f is titer of 0.1N caustic soda solution, b is titrated volume(ml) of 0.1N caustic soda solution.

The method of determination is useful for samples of both high and low molecular weight, and its use is not limited by the molecular weight. Deacetylated chitin of the present invention is meant to be a polymer, with molecular weight of at least 2,000.

The antitumor agent of the present invention may be in the form of tablets, capsules, powders, suspensions and solutions for oral administration, or in the form of ointments and poultices for external application, or in the form of injectable solutions.

The aforementioned products may be made by well known methods.

For example, for production of orally administered formulations, it will be possible to add diluting agents, adjuncts and additives as required to deacetylated chitin as the active component. The diluting agents are divided into bulking agents and augmentors, and include saccharides, starches, inorganic substances and crystalline celluloses. The adjuncts are composed of buffers, emulsifiers, dispersants, binders, lubricants, and disintegrating agents. Additives may include preservatives, aromatics and flavors.

For preparation of externally applied formulations, fat bases and emulsified bases listed in the Japanese Pharmacopeia may be used as bases.

For selection of the base, it is desirable to choose the base suitable for symptoms of the disease for which the agent is to be indicated.

For solution for preparation of the injectable solution, injectable distilled water, and/or physiological saline solution as specified in the Japanese Pharmacopela may be used for dissolution, emulsification or suspension.

It will be possible to add other drugs such as antitumor agents as required to the various formulations for additive effects.

This may be a useful means, as a variety of antitumor agents are concomitantly used in chemotherapy at present.

The volume of deacetylated chitin to be added to these formulations is not constant but may be varied according to the diseases, symptoms and types of formulations. Generally, it is within the range of 0.3 to 20 weight %, but is not limited to this range, since it is permissible to take the deacetylated chitin as a powder.

As for the mode of administration of the antitumor agent of the present invention, it may be administered orally as powders, granules, and capsules, or applied externally as externally used formulations, or by subcutaneous, intramuscular and intravenous injection.

Dosage is not constant but variable according to symptoms and types of formulations, but generally it is desirable to be within the range of 0.1 to 10g of the active constituent per day.

Indications for the treatment with the antitumor agent of the present invention include malignant tumors, more particularly tumors of blood cells, that is, hematocytes such as erythrocytes, leukocytes and platelets proliferating out of normal control, and diseases presenting symptoms of heteromorphism, maturation disorders and abnormal differentiation of blood cells.

The are a variety of diseases classified as tumors of blood cells, but are broadly classified into tumors of bone marrow cells and lymphatic cells.

Bone marrow blood cells are classified into three general classes of leukocytes (granulocytes, monocytes), erythrocytes and thrombocytes, and tumorous proliferation of each of these classes have been recognized. Leukocytic tumors include myelocytic leukemia mainly manifested as proliferation of granulocytes, myelomonocytic leukemia with proliferation of monocytes, erythrocytic leukemia and erythremia showing tumorous proliferation of erythrocytes, while thrombocytic tumors are classified as thromboblastic. More particularly, myelogenous leukemia, monocytic leukemia (myeloblastic leukemia, chronic myelocytic leukemia), polycythemia vera, erythremia, thrombocythemia, thromboblastosis, myelofibrosis, di Guglielmo syndrome and erythrocytic leukemia.

Lymphatic tumors are classified into Hodgkin's disease and non-Hodgkin's lymphoma. Lymphocytes are composed of two functionally different cells of different differentiation process, that is, T-cells and B-cells. Thus, non-HodgKin's lymphoma is further divided into B-cell lymphoma and T-cell lymphoma. The antitumor agent of the present invention has excellent effect against T-cell lymphoma and T-cell leukemia. It is particularly ef-

3

fective against adult T-cell leukemia (ATL), and Sézary's syndrome, which is malignant lymphoma of the skin. More particularly they include but not limited to Hodgkin's disease, B-cell lymphoma, non-Hodgkin's lymphoma, reticulocytic sarcoma, chronic lymphocytic leukemia, acute lymphoblastic leukemia in children, lymphoblastic lymphoma. Stenberg's sarcoma, adult T-cell leukemia, adult T-cell lymphoma, polymorphic T-cell leukemia, mycosis fungoides lymphoma, Sézary's syndrome and multiple myeloma.

Acute toxicity of this agent was investigated using samples of 70% deacetylation, $LD_{50}$ of a saline suspension administered intraperitoneally in mice as computed by Litchfield-Wilcoxon's method was 7.5g/kg or more, indicating this agent to be very safe.

The present invention will now be further explained by examples of embodiment, but the present invention will not be limited by such embodiment.

Example 1

A coarse chitin powder (a product of Shin-Nihon Chemicals) is ground to 200 mesh, treated with 1N-hydrochloric acid at 4°C for an hour, and heated at 90°C for three hours in 3% aqueous solution of caustic soda to remove calcium and proteins from the coarse chitin powder. Deacetylation of the processed chitin powder was 5.2%. Further deacetylation is carried out by heating at 121°C for an hour in a 40% aqueous solution of caustic soda, and after repeated washing and drying, a deacetylated chitin was obtained. The degree of deacetylation of the deacetylated chitin was 71.2%

The resulting chitin powder deacetylated to 71.2% was used as a powdered agent for treatment of an 80-year-old patient with Sézary's syndrome, for which an etretinate therapy was ineffective. To this patient, 0.5g/day of the deacetylated chitin powder was administered orally, and hematologic tests were performed.

The number of white blood cells and atypical lymphocytes in the blood were counted under a microscope, T4 and T8 each represents the number of T-cells counted by means of flow cytometry using, respectively, OK series OKT4 Kit (Nichirei Corp.) and Orthommune OKT Kit (Ortho Diagnostic Systems, Inc.) with monoclonal antibodies CD4 and CD8.

The results of the determination are summarized in Table 1.

Table 1

Changes in WBC count.

Atypical Lymphocyte Ratio, and T4/T8

| | WBC count | Atypical Lymphocyte Ratio | T4 | T8 | T4/T8 |
|---|---|---|---|---|---|
| Before oral administration | 11,400 | 50% | 92.9 | 3.8 | 24.5 |
| 6 months after | 6,800 | 12% | 54.5 | 12.6 | 4.3 |

The hematologic findings indicate the normalized immunologic functions as demonstrated by the decrease in T4/T8. Further, clinical symptoms improved, as WBC count and ratio of atypical lymphocytes tended to decrease from after about two months of oral administration. After about four months of treatment, symptoms of erythroderma improved, and the erythroderma marker SCC decreased.

Example 2

A coarse chitin powder (a product of Shin-Nihon Chemicals) is ground to 200 mesh, treated with 1N-hydrochloric acid at 4°C for three hours, and heated at 90°C for three hours in 3% aqueous solution of caustic soda to remove calcium and proteins from the coarse chitin powder. Deacetylation of the processed chitin powder was 6.7%. Further deacetylation is carried out by heating at 121°C for an hour in a 40% aqueous solution of caustic soda, and after repeated washing and drying, a deacetylated chitin was obtained. The degree of deacetylation of the deacetylated chitin was 85.6%.

The resulting chitin powder deacetylated to 85.6% was orally administered as a powdered agent to a patient with adult T-cell leukemia at the dose of 0.5g, three times daily. As a result, WBC count for this patient decreased significantly from 15,800 to 8,300 after about three months of administration.

Antitumor agent of the present invention demonstrated an excellent therapeutic effect against tumors of blood cells, and is very effective with little burden in the treatment of patients with these diseases, as it does not induce side effect.

**Claims**

1.   Use of one or more partially or completely deacetylated chitin derivatives or salts thereof in the manufacture of a pharmaceutical agent for the treatment of tumours of cells of the haematopoietic system.

2. Use as claimed in claim 1 wherein said chitin is deacetylated to a degree of not less than 30%.

3. Use as claimed in claim 1 or claim 2 wherein said chitin is deacetylated to a degree of 50 to 95%.

4. Use as claimed in claim 3 wherein said chitin is deacetylated to a degree of 60 to 85%.

5. Use as claimed in any one of the preceding claims wherein said deacetylated chitin has a molecular weight of at least 2000.

6. Use as claimed in any one of the preceding claims wherein said tumours of the haematopoietic system are tumours of leukocytes, erythrocytes or thrombocytes.

7. Use as claimed in any one of claims 1 to 5 wherein said tumours of the haematopoietic system are tumours of the lymphatic system.

8. A pharmaceutical composition comprising as active component a deacetylated chitin as defined in any one of claims 1 to 5.

9. A pharmaceutical composition comprising a deacetylated chitin as defined in any one of claims 1 to 5 together with at least one pharmaceutically acceptable carrier or excipient.

10. A composition comprising a deacetylated chitin, as defined in any one of claims 1 to 5 and at least one other antitumour agent in a form suitable for simultaneous or sequential administration in the treatment of tumours of the haematopoietic system.

European Patent
Office

**EUROPEAN SEARCH REPORT**

Application Number

EP 92 30 4155

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| D,X | EP-A-0 183 556 (IHARA CHEMICAL IND. CO., LTD) <br> * Abstract; page 9, line 18 - page 11, line 14; pages 33-37; examples 11-13; tables 7-9 * & JP-A-62 123 123 <br> --- | 1-4,8,9 | A 61 K 31/73 |
| X | EP-A-0 285 357 (K.K. UENO SEIYAKU) <br> * Abstract; page 5, lines 11-24; page 7, lines 22-27; page 8, line 51 - page 9, line 8; example 1; page 14, table 3; claim 20 * <br> --- | 1-9 | |
| X | EP-A-0 240 098 (K.K. UENO SEIYAKU) <br> * Abstract; page 14, table 3; claims 1-5,9,22 * <br> --- | 1-9 | |
| X | DIALOG INFORMATION SERVICES INC., FILE: CHEMICAL ABSTRACTS, accession no: 114049603, T. UENO et al.: "Suppositories containing sulfo group-containing oligo- or polysaccharides for treating AIDS and other diseases" <br> * Whole document * & PATENT ABSTRACTS OF JAPAN, vol. 14, no. 453 (C-764)[4396], 28th September 1990; & JP-A-2 178 230 (UENO SEIYAKU OYO KENKYUSHO K.K.) 11-07-1990 <br> --- | 1-9 | **TECHNICAL FIELDS SEARCHED (Int. Cl.5)** <br><br> A 61 K |
| X | VACCINE, vol. 2, no. 1, March 1984, pages 93-99; K. NISHIMURA et al.: "Immunological activity of chitin and its derivatives" <br> * Abstract; page 93, column 2, last paragraph - page 94, column 1, paragraph 1 * <br> ---        -/- | 8,9 | |

The present search report has been drawn up for all claims

| Place of search | Date of completion of the search | Examiner |
|---|---|---|
| THE HAGUE | 13-07-1992 | MAIR J. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)

**European Patent Office**

**EUROPEAN SEARCH REPORT**

Application Number

EP  92 30 4155

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| A | * Whole document *  <br>--- | 1-7,10 | |
| X | VACCINE, vol. 3, no. 5, December 1985, pages 379-384; K. NISHRA et al.: "Adjuvant activity of chitin derivatives in mice and guinea-pigs" <br>* Abstract; page 379, column 2, paragraph 3 * <br>--- | 8,9 | |
| A | * Whole document * <br>--- | 1-7,10 | |
| X | JOURNAL OF BIOACTIVE AND COMPATIBLE POLYMERS, vol. 4, no. 4, October 1989, pages 362-371; T. OUCHI et al.: "Antitumor activity of chitosan and chitin immobilized 5-fluorouracils through hexamethylene spacers via carbamoyl bonds" <br>* Whole document * <br>----- | 1-10 | |
| | | | TECHNICAL FIELDS SEARCHED (Int. Cl.5) |

The present search report has been drawn up for all claims

| Place of search | Date of completion of the search | Examiner |
|---|---|---|
| THE HAGUE | 13-07-1992 | MAIR J. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)